# FASCICULE DE BREVET EUROPEEN

(11) **EP 2 419 115 B1**
(45) Date de publication et mention de la délivrance du brevet: **31.12.2014**
(21) Numéro de dépôt: 10719385.6
(22) Date de dépôt: 15.04.2010
(51) Int. Cl.: A23L 1/30, A61K 36/185, A61K 36/03, A61K 36/064, A61K 31/20, A61K 33/30, A61K 33/26, A61K 45/06

(54) **COMPOSITION DESTINEE A LA REGULATION DU METABOLISME DES LIPIDES**
ZUSAMMENSETZUNG ZUR REGULIERUNG DES LIPID-STOFFWECHSELS
COMPOSITION FOR REGULATING THE METABOLISM OF LIPIDS

(30) Priorité: 15.04.2009 WO PCT/IB2009/005239
(43) Date de publication de la demande: 22.02.2012
(73) Titulaire: Actigenomics S.A., 1066 Epalinges (CH)
(72) Inventeur: WAHLI, Walter, CH-1112 Echichens (CH); BOURGEOIS-LUGAND, Marie Françoise Yvonne, F-29530 Loqueffret (FR); HUSSON-ROBERT, Bernadette, F-21850 Saint Apollinaire (FR); PARISOT, Gilles, Didier, CH-1867 Ollon (CH)
(74) Mandataire: KATZAROV S.A.
(86) Numéro de dépôt international: PCT/IB2010/051653
(87) Numéro de publication internationale: WO 2010/119424

(56) Documents cités:
- EP-A1- 1 857 112
- EP-A1- 2 036 444
- WO-A1-2004/105770
- WO-A1-2009/050580
- WO-A2-2006/016363
- PLAT J ET AL: "EFFECTS OF PLANT STEROLS AND STANOLS ON LIPID METABOLISM AND CARDIOVASCULAR RISK", NMCD. NUTRITION METABOLISM AND CARDIOVASCULAR DISEASES, MILAN, IT, vol. 11, no. 1, 1 février 2001 (2001-02-01), pages 31-40, XP008037504, ISSN: 0939-4753

## Description

### Domaine de l'invention

La présente invention concerne une composition nutraceutique et/ou alimentaire destinée entre autre à la régulation du métabolisme des lipides chez l'Homme ou l'Animal ainsi que des méthodes d'utilisations de ladite composition nutraceutique et/ou alimentaire.

### Discussion de l'état de la technique

Les thérapies diététiques deviennent incontournables dans la régulation du métabolisme lipidique et plus particulièrement pour provoquer une diminution du LDL-cholestérol et réduire les risques des maladies cardiaques coronariennes. Dans ce but, dans de nombreux pays, de la nourriture enrichie en stérols et stanols végétaux a été développée, en parallèle à l'évolution mondiale des maladies coronariennes, causes essentielles de morbidité et de mortalité.

### Phytosterols:

Les phytosterols (stérols et stanols issus des plantes) sont une option diététique disponible pour réduire le cholestérol plasmatique (TC) et le LDL-cholestérol, depuis que ces effets biochimiques ont été montrés chez l'Homme dans les années 1950. Depuis, de nombreuses études cliniques ont conforté les résultats : principalement les stérols et stanols estérifiés à partir des huiles végétales (essentiellement des C18) diminuent significativement le CT et les LDL-cholestérol lorsqu'ils sont administrés dans la nourriture (Katan et al, 2003). 2g/jour de phytostérols abaissent de 10% les concentrations de LDL-cholestérol.

Plus de 40 stérols végétaux ont été identifiés, le sitostérol, le campestérol et le stigmastérol sont les plus abondants. Les stanols (sitostanol et campestanol) sont des stérols saturés et sont moins répandus dans la nature que les stérols.

Alors que 50% du cholestérol est absorbé dans le tractus intestinal, l'absorption intestinale des stanols et des stérols est beaucoup plus réduite : 10-15% pour le campestérol et le campestanol, 4-7% pour le sitostérol et 1% pour le sitostanol. Les stanols et les stérols sont hydrolysés dans l'intestin grêle supérieur.

Le mécanisme d'action principal réduisant le taux de cholestérol avec les phytostérols et phytostanols est l'inhibition de l'absorption intestinale de cholestérol, faisant intervenir les protéines de la superfamille des « ATP-binding cassette » (ABC) G, plus particulièrement ABCG5 et ABCG8. La dose journalière recommandée de phytostérols pour obtenir une réduction de 30 à 40% de l'absorption intestinale de cholestérol est de 2g (Katan et al, 2003, Trautwein et al, 2003), sachant que la dose ingérée par jour de stérols ou stanols végétaux varie de 0.150 à 0.450mg/jour.

L'estérification du sitostanol ou du sitostérol avec les acides gras augmente à la fois leur solubilité dans les mayonnaises et les margarines et aussi leur dispersion intestinale, optimisant ainsi leur efficacité.

La forme physique des stérols et stanols est très importante; les stanols et stérols libres (ie non estérifiés) peuvent avoir les mêmes effets sur les lipoprotéines plasmatiques que les esters (Katan et al, 2003) ; toutefois la matrice et le procédé d'émulsification sont très importants et peuvent apporter des résultats négatifs (Denke, 1995). Les stanols libres, émulsifiés avec la lécithine, réduisent l'absorption intestinale de 37% lors d'une administration unique. Il n'y a pas de modification importante de l'efficacité selon la dissolution des stérols/stanols dans le diacylglycérol ou leur incorporation dans des produits basses calories (pain, céréales ou yogourts basses calories) ; même si l'efficacité est moins bonne avec le pain et les céréales. Cependant les effets sur les lipoprotéines des diméthylstérols du son de riz sont plus faibles que ceux des stérols provenant du cholestérol tel que le sitostérol.

Dans de nombreuses études cliniques, la prise journalière (2.5g) des stérols ou stanols est répartie en 2 ou 3 portions, ou bien en une prise au déjeuner pour donner le même effet sur la réduction du LDL-cholestérol. La distribution de la prise des stérols ou stanols n'est pas déterminante pour leur efficacité, seule compte la quantité par jour. Les stérols et stanols doivent abaisser les taux de cholestérol, aussi bien chez des personnes sous régime normal que chez des personnes sous diète hyper riche. Ainsi les phytostérols/stanols sont des compléments pour les diètes saines faiblement concentrées en acides gras saturés et en cholestérol mais aussi dans les diètes enrichies en fruits, légumes et huiles comme sources de graisses.

### Effets secondaires:

Les stanols et les stérols ne présentent pas d'effets indésirables très sérieux et donc la balance risques/bénéfices apparait nettement favorable.

Cependant, depuis que les stérols et stanols ont été trouvés dans la plaque d'athérome, il a été suggéré que l'athérosclérose primaire chez les patients avec une phytostérolémie homozygote est due à un effet athérogénique des sitostérols et campestérols circulants. Toutefois le taux élevé d'athérosclérose chez ces patients phytostérolémiques peut être dû à d'autres désordres génétiques.

Quelques expérimentations rapportent que les stérols et stanols végétaux ont des effets oestrogéniques, toutefois les stérols ne se lient pas aux récepteurs des oestrogènes. 18 études cliniques ont testé la dose de 1.5g/jour de stanols/stérols et ont montré une réduction significative des concentrations plasmatiques de caroténoïdes dont le α carotène (- 9%), β carotène (-28%), lycopène (-7%). La diminution du β carotène peut être évitée en consommant des fruits ou des légumes compensateurs en caroténoïdes (Clifton et al., 2004).

Par ailleurs, il est connu que les nutriments tels que les oligoéléments, les plantes, les principes alimentaires (acides aminés) ou les vitamines peuvent activer ou inhiber certaines fonctions de l'organisme. La micronutrition, consistant à apporter à l'organisme un ou plusieurs nutriments en quantités réduites, a permis de résoudre partiellement les problèmes posés par la nutrition classique, dont l'encombrement des sites d'absorption au niveau intestinal. En effet, les microquantités de nutriments administrés ne saturent pas les sites d'absorption intestinale, et les nutriments absorbés sont alors directement assimilables par les cellules.

En particulier, WO 2009/050580 (Bioresearch & Partners) décrit une composition, ci-après définie sous l'appellation composition LIPISTASE, destinée à la régulation du métabolisme des lipides et des méthodes utilisables dans le domaine de l'alimentation ainsi que le domaine nutraceutique et thérapeutique. Elle concerne notamment des additifs ou compléments alimentaires, une composition les contenant et leurs utilisations, notamment pour revitaliser le métabolisme de sujets, notamment humains. Malgré les solutions proposées par l'art antérieur, il n'existe cependant pas de composition ou additif alimentaire efficace, ne présentant pas ou peu d'effet indésirable ou secondaire pour l'organisme et qui permette d'agir à la fois sur:
- Les effets secondaires induits par les grandes quantités de PS administrées ainsi que leur fréquence d'administration ;
- Les dyslipidémies familiales et liées au syndrome métabolique ;
- La prévention du syndrome métabolique et du pré-diabéte de type 2 ;
- Les maladies cardiovasculaires, certains cancers et la dégénérescence maculaire ;
- L'hypertension et la thrombose, l'athérosclérose ;
- Les maladies neuro dégénératives, comme par exemple la maladie d'Alzheimer.

### Brève description de l'invention

La présente invention, se propose de répondre à cette demande par l'élaboration d'une composition nutraceutique et/ou alimentaire innovante destinée, entre autre, à la régulation du métabolisme des lipides chez l'Homme ou l'Animal.

La présente invention concerne plus particulièrement une composition nutraceutique et/ou alimentaire comprenant:
- 7µg à 700µg (par 100g/100ml) d'au moins deux huiles végétales sélectionnées parmi l'huile de colza, l'huile d'olive, l'huile de pépins de raisin et l'huile d'onagre,
- 10µg à 1000µg (par 100g/100ml) de minéraux chargés positivement choisis parmi le sodium, le magnésium et le calcium,
- 10µg à 100µg (par 100g/100ml) de métaux choisis parmi le zinc et le fer,
- 7µg à 700µg (par 100g/100ml) de levures ou extraits de levure provenant du genre *Saccharomyces cerevisiae,* caractérisé en ce que ces dites levures ou extraits de levure sont enrichies en sélénium,
- 7µg à 700µg (par 100g/100ml) de champignons ou d'extraits de champignon shiitaké (mycelium)
- 6µg à 600µg (par 100g/100ml) d'au moins deux extraits végétaux de plante choisis parmi la criste marine, l'ail et la vigne,
- 8µg à 800µg (par 100g/100ml) d'au moins une vitamine choisie parmi les vitamines A, B1, B9, C, E, F et PP
- 7µg à 700µg (par 100g/100ml) d'huile animale et de coprah (*Cocos nucifera*)
- 6µg à 600µg (par 100g/100ml) d'au moins une algue choisie parmi Palmaria palmata (Dulse), Chondrus crispus (Carragaeen) et Fucus vesiculosus (Varech vésiculeux),
en combinaison avec un ou plusieurs phytostérols, stanols ou leurs mélanges ainsi qu'un excipient pharmaceutique autorisé.

Le ou les phytostérols/stanols (désignés ici sous les initiales PS) et/ou mélanges ainsi que ledit excipient complèteront avantageusement le volume de manière à obtenir les 100 ml de la composition pharmaceutique selon l'invention.

Il a été démontré que la composition nutraceutique et/ou alimentaire selon l'invention produit des effets synergiques et de loin supérieurs à ceux de la composition LIPISTASE seule (WO 2009/050580; Bioresearch & Partners) ou les phytostérols/stanols pris isolement.

D'autres objets de l'invention concernent des méthodes :
- pour la régulation de l'homéostasie lipidique
- pour la prévention du syndrome métabolique, des risques de cancers, des maladies cardiovasculaires, de la dyslipidémie, de l'hypertension, de la progression de l'athérosclérose, de l'obésité, de l'hyperglycémie, de la dégénérescence maculaire du diabétique, des maladies neuro dégénérative chez l'Homme et l'Animal.

L'utilisation de la dite composition nutraceutique et/ou alimentaire pour la préparation d'un médicament destiné au traitement et/ou à la prévention du syndrome métabolique, des risques de cancers, des maladies cardiovasculaires, de la dyslipidémie, de l'hypertension, de la progression de l'athérosclérose, de l'obésité, de l'hyperglycémie, de la dégénérescence maculaire du diabétique, des maladies neuro dégénérative ainsi que la régulation de l'homéostasie lipidique chez l'Homme et l'Animal est également un des objets de la présente invention.

D'autres avantages inattendus de la composition nutraceutique et/ou alimentaire selon l'invention apparaîtront à la lecture de la description détaillée et des exemples de réalisation de l'invention.

### Brève description des figures

**Figure 1** **:** Evolution du poids corporel des souris C57B16J soumises au régime enrichi en cholestérol et sous différents traitements, exprimée en g, * p<0.05, n=12.
**Figure 2** **:** Gain de poids des souris C57B16J soumises au régime enrichi en cholestérol et effet des différents traitements, exprimé en g, * p<0.05, n=12
**Figure 3** **:** Prise alimentaire, consommation en g par jour et par souris, n=12
**Figure 4** **:** Prise alimentaire cumulée par souris et au bout de 12 semaines de traitement, exprimée en g.
**Figure 5** **:** Index d'adiposité après 12 semaines de traitement, exprimé en % et pourcentages de variation par rapport aux contrôles HC (diète enrichie en cholestérol), ** p <0.01, n=12.
**Figure 6** **:** Répartition du tissu adipeux blanc après 12 semaines de traitement, exprimé en g, et pourcentages de variation par rapport aux contrôles HC (diète enrichie en cholestérol), ** p <0.01, * p<0.05, n=12.
**Figure 7** **:** Coupes histologiques de tissu adipeux sous cutané après 12 semaines de traitement, coloration HE, grossissement x20.
**Figure 8a** **:** Morphométrie des tissus adipeux, périmètres des adipocytes périrénaux et sous cutanés, pourcentages de variation par rapport aux contrôles HC (diète enrichie en cholestérol), * p<0.05, n=12.
**Figure 8b** **:** Morphométrie des tissus adipeux, surfaces des adipocytes périrénaux et sous cutanés, pourcentages de variation par rapport aux contrôles HC (diète enrichie en cholestérol), * p<0.05, n=12.
**Figure 9** **:** Index de sécrétion d'adiponectine [concentration adiponectinémie (µg/ml)/masse du tissus adipeux (g)], pourcentages de variation par rapport aux contrôles HC (diète enrichie en cholestérol), ** p <0.01, * p<0.05, n=12.
**Figure 10a** : Evolution de la cholestérolémie non à jeun, pourcentages de variation par rapport aux contrôles HC (diète enrichie en cholestérol), ** p <0.01, * p<0.05, n=12.
**Figure 10b** : Evolution de la cholestérolémie après 4h de jeûne, pourcentages de variation par rapport aux contrôles HC (diète enrichie en cholestérol), ** p <0.01, * p<0.05, n=12.
**Figure 11a** **:** Répartition du cholestérol total dans les lipoprotéines, pourcentages de variation par rapport aux contrôles HC (diète enrichie en cholestérol), *** p <0.001, ** p <0.01, * p<0.05, n=12.
**Figure 11b** **:** Répartition de cholestérol libre dans les lipoprotéines, pourcentages de variation par rapport aux contrôles HC (diète enrichie en cholestérol), *** p <0.001, ** p <0.01, * p<0.05, n=12.
**Figure 11c** **:** Répartition de l'ester de cholestérol dans les lipoprotéines, pourcentages de variation par rapport aux contrôles HC (diète enrichie en cholestérol), *** p <0.001, ** p <0.01, * p<0.05, n=12.
**Figure 12** **:** Evolution de la triglycéridémie après 4h de jeûne, pourcentages de variation par rapport aux contrôles HC (diète enrichie en cholestérol), n=12.
**Figure 13** **:** Quantification des glycémies non à jeun après 12 semaines de traitement, n=12
**Figure 14** **:** Quantification des insulinémies non à jeun après 12 semaines de traitement, pourcentages de variation par rapport aux contrôles HC (diète enrichie en cholestérol), n=12.
**Figure 15** **:** Mesure des ALAT sur sérum d'animaux non à jeun après 12 semaines de traitement, pourcentages de variation par rapport aux contrôles HC (diète enrichie en cholestérol), n=12.
**Figure 16** **:** Mesure des ASAT sur sérum d'animaux non à jeun après 12 semaines de traitement, pourcentages de variation par rapport aux contrôles HC (diète enrichie en cholestérol), *** p <0.001, n=12.
**Figure 17** **:** Coupes histologiques du lobe médian de foie après 12 semaines de traitement, coloration aux huiles rouges pour marquage des lipides, grossissement X 20.
**Figure 18** **:** Ratio poids du foie/poids du corps en pourcentage après 12 semaines de traitement, n=12.

### Description détaillée de l'invention

La présente invention relate de la mise au point d'une composition nutraceutique et/ou alimentaire, ayant des propriétés particulièrement avantageuses sur la régulation du métabolisme des lipides chez l'Homme ou l'Animal.

Un premier objet de la présente invention réside dans une composition nutraceutique et/ou alimentaire, comprenant la combinaison de la composition LIPISTASE :
- 7µg à 700µg (par 100g/100ml) d'au moins deux huiles végétales sélectionnées parmi l'huile de colza, l'huile d'olive, l'huile de pépins de raisin et l'huile d'onagre,
- 10µg à 1000µg (par 100g/100ml) de minéraux chargés positivement choisis parmi le sodium, le magnésium et le calcium,
- 10µg à 1000µg (par 100g/100ml) de métaux choisis parmi le zinc et le fer,
- 7µg à 700µg (par 100g/100ml) de levures ou extraits de levure provenant du genre Saccharomyces cerevisiae, caractérisé en ce que ces dites levures ou extraits de levure sont enrichies en sélénium,
- 7µg à 700µg (par 100g/100ml) de champignons ou d'extraits de champignon shiitaké (mycelium)
- 6µg à 600µg (par 100g/100ml) d'au moins deux extraits végétaux de plante choisis parmi la criste marine, l'ail et la vigne,
- 8µg à 800µg (par 100g/100ml) d'au moins une vitamine choisie parmi les vitamines A, B1, B9, C, E, F et PP
- 7µg à 700µg (par 100g/100ml) d'huile animale et de Coprah (Cocos nucifera)
- 6µg à 600µg (par 100g/100ml) d'au moins une algue choisie parmi Palmaria palmata (Dulse), Chondrus crispus (Carragaeen) et Fucus vesiculosus (Varech vésiculeux),
en combinaison avec un ou plusieurs phytostérols, stanols ou leurs mélanges ainsi qu'un excipient pharmaceutique autorisé.

Le ou les phytostérols/stanols (PS) ainsi que ledit excipient complèteront avantageusement le volume de manière à obtenir les 100 ml de la composition pharmaceutique selon l'invention.

On utilisera avantageusement une quantité légale de phytostérols et ou de stanols selon l'intervalle autorisé compris entre 1.6 et 3.3 g/jour. La quantité recommandée de phytostérols et ou stanols ou leur mélange est préférablement comprise entre 2 et 2.4 g/jour (I Demonty, RT Ras, HCM Van der Knaap, G Duchateau, L Meijer, PL Zock, JM Geleijnse, EA Trautwein, « continuous dose-response relationship of the LDL-cholesterol-lowering effect of phytosterol intake" JNutr 139, 271-284, 2009.).

Dans un mode de réalisation particulier de l'invention, les PS utilisés sont de préférence sélectionnés parmi le groupe comprenant : le brassicastérol, campestérol, campestanol, stigmasterol, beta-sitosterol, beta sitostanol, D5 avenastérol, D7 stigmasténol, D7Avenastérol ou leur combinaisons et mélanges. De préférence on utilisera un mélange de ces PS cités ci-dessus.

Selon un mode préféré de l'invention, la préparation de PS (phytostérols et stanols) sera avantageusement constituée dans les proportions suivantes : cholestérol 0.4%, brassicastérol 3.2%, campestérol 27.2%, campestanol 0.7%, stigmasterol 15.4%, beta-sitosterol 47%, beta sitostanol 1%, D5 avenastérol 1.9%, D7 stigmasténol 0.4%, D7Avenastérol 0.2%, stérols non identifiés 2.6%.

De manière préférée, la composition nutraceutique et/ou alimentaire selon l'invention comprend de l'huile de poisson des mers froides (*oleum pisci mare fresca*) en tant qu'huile animale.

Selon un mode de réalisation préféré, la composition nutraceutique et/ou alimentaire selon l'invention contient au moins deux vitamines choisies parmi les vitamines A, B1, B9, C, E, F et PP.

Plus particulièrement, la composition nutraceutique et/ou alimentaire selon l'invention comprend préférablement de :
- 7µg à 700µg (par 100g/100ml) d'huile de colza, de l'huile d'olive, de l'huile de pépins de raisin(s), de l'huile d'onagre,
- 10µg à 1000µg (par 100g/100ml) de sodium, de magnésium et de calcium,
- 10µg à 1000µg (par 100g/100ml) de zinc et le fer,
- 7µg à 700µg (par 100g/100ml) de levures ou extraits de levure de Saccharomyces cerevisiae, enrichis en sélénium,
- 7µg à 700µg (par 100g/100ml) de mycélium ou d'extrait de mycélium de Shiitake,
- 6µg à 600µg (par 100g/100ml) de criste marine, d'ail et de vigne,
- 8µg à 800µg (par 100g/100ml) de vitamines A, B1, B9, C, E, F et PP
- 7µg à 700µg (par 100g/100ml) d'huile de poisson des mers froides et de Coprah,
- 6µg à 600µg (par 100g/100ml) de Palmaria palmata (Dulse), Chondrus crispus (Carragaeen) et Fucus vesiculosus (Varech vésiculeux)
en combinaison avec un ou plusieurs phytostérols, stanols ou leurs mélanges ainsi qu'un excipient pharmaceutique autorisé.

La composition nutraceutique et/ou alimentaire de l'invention peut de préférence comprendre des excipients ou additifs, comme par exemple : eau, huile, lactose-saccharose ou lactose-amidon, fructo-oligosaccharides, sorbitol, phosphate dicalcique ou parmi les excipients utilisés en alimentaire (en tant qu'additifs alimentaires) : colorants, conservateurs (sorbate de potassium, benzoate de sodium), arômes, antioxydants (caroténoïdes, vitamines C et E, flavonoïdes), émulsifiants (lécithine, mono et diglycérides d'acides gras), stabilisants et gélifiants (lécithine, lactate de potassium, agar agar, carraghénanes, alginate de sodium), exhausteurs de goût (sels de l'acide glutamique, inosinate de sodium), acidifiants (acide citrique, maltate de sodium), anti-agglomérants (stéarate de magnésium, dioxyde de silicium), édulcorants (sorbitol, sodium saccharinate).

De manière préférentielle, la composition selon l'invention comprend aux moins deux huiles choisies parmi l'huile de colza, l'huile d'olive, l'huile de pépins de raisin(s) et l'huile d'onagre. Avantageusement, la composition comprend au moins trois huiles végétales, plus préférentiellement elle comprend l'huile de colza, l'huile d'olive, l'huile de pépins de raisin(s) et l'huile d'onagre.

L'huile de colza (*oleum Brassica napus oleifera*) est constituée de 98% de triesters d'acides gras ; les 2% restants sont riches en stérols et tocophérols (dont la vitamine E). C'est une huile riche en acide alpha-linolénique, acides gras poly-insaturés oméga 3, en acides gras mono-insaturés oméga 6 (avec un rapport intéressant oméga3:6 de 1:2,5) ; et seulement 6 à 8% d'acides gras saturés.

L'huile d'olive (oleum Olea europea) est une huile riche en acide oléique : acide gras mono-insaturés (supérieur à 75%), oméga 6 (8%); l'huile d'olive contient des vitamines A, E et K. Le rapport vitamine E / AGPI (Acides Gras Poly Insaturés) est le plus élevé de toutes les huiles.

L'huile de pépins de raisin (oleum Vitis vinifera) est une huile équilibrée en acide linoléique (alpha-linoléique et béta-linoléique), en acides oléique, palmitique et stéarique. Cette huile contient plus de 70% d'Omega 6. Elle est fortement insaturée : le rapport poly-insaturés/saturés supérieur à 5.

L'huile d'onagre (Oleum Oenothera biennis) est une huile Oméga 6 équilibrée en acide linoléique, en acide gamma-linolénique, en acides oléique et stéarique.

Dans un mode de réalisation particulier, la composition nutraceutique et/ou alimentaire selon l'invention comprend de l'huile de colza, de l'huile d'olive, de l'huile de pépins de raisin(s) et de l'huile d'onagre.

De manière préférée, les minéraux comprennent un ou plusieurs minéraux chargés positivement, choisis de préférence parmi le sodium, le magnésium et le calcium.

Le sodium permet la régulation acide-base de l'organisme et du métabolisme cellulaire. Il joue un rôle déterminant dans la dépolarisation cellulaire qui est à l'origine de l'excitabilité et de la conduction des influx (en particulier neuromusculaire et cardiaque), dans le maintien de l'équilibre acido-basique, de la pression osmotique, dans l'équilibre des échanges liquidiens et ioniques de l'organisme.

Le magnésium est indispensable à l'équilibre des canaux ioniques. Il agit comme cofacteur enzymatique et module les systèmes de transport du sodium (Na⁺) et du potassium (K⁺) dans tous les tissus ; il est le régulateur physiologique du calcium dans l'équilibre des échanges cellulaires. Le magnésium joue un rôle de stabilisateur des différents organites intracellulaires : il stabilise les ribosomes qui produisent les protéines, maintient la production d'énergie par les mitochondries car indispensable à la synthèse des molécules d'ATP. Cette production d'énergie est la base de tous les mécanismes de vie cellulaire et de la vitalité globale de l'organisme. Le magnésium est indispensable à la synthèse des protéines fondamentales à la construction cellulaire (certains acides aminés, ADN et ARN).

Le calcium est impliqué dans de nombreuses réactions enzymatiques. Il permet la transmission des informations au niveau cellulaire en tant que second messager ce qui induit la transmission de l'influx nerveux, la contraction musculaire (par le déplacement des fibres d'actine et de myosine), la stimulation de sécrétions hormonales par certaines cellules (sécrétion d'insuline par les cellules β pancréatiques par exemple...) et la libération des neuromédiateurs dans le système nerveux central.

Dans un mode de réalisation particulier, la composition nutraceutique et/ou alimentaire selon l'invention comprend du sodium, du magnésium et du calcium.

De manière préférée, les métaux comprennent un ou plusieurs métaux choisis parmi le zinc et le fer.

Le zinc intervient dans l'activité de près de 200 enzymes [en particulier dans des systèmes enzymatiques comme les oxydoréductases, l'alcool déshydrogénase, la cytochrome réductase et la SOD (superoxydismutase)]. Les enzymes concernées par le zinc ont une importance métabolique considérable : la glycolyse, la voie des pentoses, la néoglucogenèse, le métabolisme des lipides, et des acides gras.

Le zinc est un métal activateur de la plupart des coenzymes nécessaires au métabolisme énergétique. Il joue un rôle très important dans l'équilibre acido-basique (anhydrase carbonique), dans l'inflammation, dans la différenciation cellulaire, dans la défense endogène anti-radicalaire. C'est aussi un cofacteur hormonal (hormone de croissance, thyroïde, corticosurrénales) et il est indispensable à la transcription de la chaîne d'ADN (ARN-polymérase).

Le zinc stabilise les membranes cellulaires en se couplant avec les groupements thiols en leur évitant ainsi de réagir avec le fer évitant de produire du peroxyde d'hydrogène (H₂O₂) radical libre très instable. Il intervient particulièrement dans le métabolisme de la vitamine A (mobilisation au niveau du foie, formation du rétinol).

Il stabilise les structures protéiques et joue un rôle dans l'expression des gènes.

Composant des cytochromes, le fer est indispensable à la détoxication et à la fabrication des hormones thyroïdiennes. De plus, il fait corps avec les sites actifs de certaines protéines dont le rôle est vital dans l'organisme : l'hémoglobine, la myoglobine, et les cytochromes.

Dans un mode de réalisation particulier, la composition nutraceutique et/ou alimentaire selon l'invention comprend du zinc et du fer.

De manière préférée, les levures ou extraits de levure sont des levures du genre Saccharomyces, ou des extraits de telles levures (par exemple des préparations membranaires, vésiculaires, protéiques, etc...). Il s'agit tout particulièrement de levures (ou extraits) du genre Saccharomyces cerevisiae.

Dans un mode particulier de réalisation, on utilise des (extraits de) levures enrichies en sélénium, qui possède des propriétés anti-oxydantes.

D'autres levures du genre Saccharomyces ou autres (Aspergillus, Torulaspora...) peuvent également être utilisées comme par exemple:
saccharomyces boulardii (utilisée en industrie agro-alimentaire)
saccharomyces cerevisiae(« fermentation haute » pour le vin, la bière),
saccharomyces uvarum (« fermentation basse » pour des bières type Lager)
schizosaccharomyces pombe (dans la bière d'Afrique)
aspergillus (saké) induisant une fermentation alcoolique
torulaspora delbrueckii et candida stellata (initialement présentes dans le moût) permettant une augmentation des esters et réduction de la formation d'acidité volatile

De manière préférée, des extraits végétaux comprennent un ou plusieurs extraits de plante(s). Plus préférentiellement, la composition selon l'invention comprend, à titre d'extrait végétal, au moins deux extraits végétaux choisis parmi la criste marine, l'ail et la vigne.

Avantageusement, la composition nutraceutique et/ou alimentaire selon l'invention comprend la criste marine, l'ail et la vigne.

Le terme "extrait" végétal désigne au sens de la présente invention toute préparation obtenue à partir de tout ou partie du végétal considéré. Il peut s'agir d'un broyat, d'un filtrat, de graines, de feuilles, de tiges, d'écorce, etc., ou de combinaisons. L'extrait peut être préparé par des techniques classiques.

La criste marine (*Crithmum maritimum)* est très riche en minéraux : zinc, fer, magnésium, cuivre et manganèse, en vitamines A, E, B1, B2. Elle présente une action détoxifiante.

L'ail (Allium sativum) est riche en vitamine C, en zinc, en manganèse, et possède une action hypocholestérolémiante. L'ail se caractérise par la présence de substances soufrées originales (trisulfure d'allyle, ajoène E) ayant des effets bénéfiques sur la fluidité sanguine (réduction de l'agrégation plaquettaire) et le taux de cholestérol sanguin (diminution de la synthèse des triglycérides) : intérêt sur le plan cardio-vasculaire. D'autres propriétés lui sont attribuées : antibactériennes, antifongiques, antivirales. Les composés organosulfurés inhibent l'activation carcinogène et participent aux développements de résistance multidrogues. Une protection hépatique et rénale contre le stress oxydatif est également rapportée.

Le raisin ou vigne (Vitis vinifera) est très riche en vitamines A et B et en sels minéraux : manganèse, potassium, calcium. Le raisin, riche en substances anti-radicalaires, facilite la détoxification / épuration par la vésicule biliaire et le foie.

La composition selon l'invention comprend au moins une algue ou un extrait d'algue choisi parmi parmi Palmaria palmata (Dulse), Chondrus crispus (Carragaeen) et Fucus vesiculosus (Varech vésiculeux).

La Dulse (Palmaria palmata) est très riche en provitamine A, (puissant anti-oxydant, renforçant les défenses immunitaires et réduisant le développement de tumeurs) et en vitamine C (antioxydant, requise dans la synthèse du collagène, des hématies, stimule le système immunitaire, promoteur de l'absorption du fer). Riche en acides aminés essentiels et en métaux [cuivre(Cu²⁺), nickel (Ni²⁺), cadmium (Cd ²⁺) et zinc (Zn²⁺)].

Le varech vésiculeux (Fucus vesiculosus) est riche en fucostérol : stérol qui présente des propriétés hypocholestérolémiantes, comme le béta-sitostérol des végétaux. Il est également très riche en iode et en fer.

Le Carragaeen (Mousse d'Irlande - Chondrus Crispus) est riche en acides gras et équilibré entre les acides gras oméga 3 et oméga 6 ainsi qu'en acides gras insaturés permettant l'assimilation du cholestérol. Il est également riche en acides aminés et en oligo-métaux (particulièrement iode, zinc et fer); et en vitamine D.

Dans un mode de réalisation particulier, la composition comprend des extraits de Palmaria palmata (Dulse), de Chondrus crispus (Carragaeen) et de Fucus vesiculosus (Varech vésiculeux).

La composition pharmaceutique selon l'invention comprend au moins un champignon ou extrait de champignon shiitaké (mycélium). Le mycélium de shiitake (Lentinus edodes) est très riche en acides aminés, oligo-éléments et vitamines. Il présente des propriétés hypocholestérolémiantes et stimulantes du système immunitaire.

De manière préférée, les vitamines comprennent une ou plusieurs vitamines choisies parmi les vitamines A, B1, B9, C, E, F et PP. Dans un mode préféré, la composition selon l'invention comprend au moins deux vitamines différentes, plus préférentiellement au moins trois, quatre, cinq ou six vitamines différentes, choisies parmi les vitamines indiquées ci-dessus.

La vitamine A (rétinol) est estérifiée dans l'entérocyte, incorporée aux chylomicrons, puis excrétée dans la lymphe d'où elle rejoint la circulation générale par le canal lymphatique. La vitamine A stabilise les membranes cellulaires, la biosynthèse et la régulation des hormones stéroïdes. Ses effets sur les cellules en bâtonnet de la rétine sont essentiels à la vision. La synthèse de certaines protéines est également sous la dépendance de la vitamine A.

La vitamine B1 (thiamine) augmente la formation intracellulaire du NADPH2, dont le rôle est primordial pour la synthèse des lipides, stérols et acides gras à partir des glucides. Elle est indispensable aux fonctionnements des systèmes nerveux (dégradation de l'acide pyruvique) et musculaire.

La vitamine B9 (acide folique) est un coenzyme participant à la synthèse des purines et pyrimidines, constituants les acides nucléiques (ADN et ARN). Cette vitamine intervient également dans la synthèse d'acides aminés (méthionine, histidine, sérine).

La vitamine C (acide ascorbique), cofacteur enzymatique, est requise dans la synthèse du collagène et des hématies, et contribue au système immunitaire. Elle favorise l'absorption du fer et son métabolisme. Passant dans le cerveau sous forme oxydée, elle est un anti-oxydant et diminue les radicaux libres. Elle intervient dans le métabolisme des lipides, en association avec diverses hydroxylases. Les hydroxylases dépendantes du cytochrome P 450 microsomal catalysent en présence de l'acide ascorbique la transformation du cholestérol en acides biliaires.

La vitamine E (alpha-tocophérol) accompagne les chylomicrons dans les canaux lymphatiques jusqu'à la circulation générale. Dans le plasma l'alpha tocophérol est lié aux diverses catégories de lipoprotéines : les LDL en portent 40 à 60%, les HDL 35 %. Le taux de vitamine E est étroitement corrélé à celui des lipides totaux et celui du cholestérol. Elle présente un effet antioxydant et réduit les radicaux libres. Elle participe à la formation et à la structure des phospholipides membranaires et par ce fait possède une action stabilisatrice sur les membranes cellulaires.

La vitamine F fait partie des vitamines liposolubles et est composée d'acides gras polyinsaturés dont essentiellement l'acide linoléique. Cet acide gras (précurseur de certaines molécules anti-inflammatoires) est indispensable pour la synthèse du cholestérol et de certains autres acides gras et participe au maintien de l'intégrité des membranes cellulaires. Ainsi, la vitamine F a un rôle crucial dans la formation de la barrière lipidique de l'épiderme.

La vitamine PP [Vitamine B3, nicotinamide (niacine)], précurseur du NAD et du NADP (2 cofacteurs intervenant dans les réactions d'oxydo-réduction), est essentielle au métabolisme des glucides, des lipides et des protéines. Elle joue un rôle dans la formation des globules rouges, la circulation sanguine, le transport de l'oxygène aux cellules, le fonctionnement du système digestif et du système nerveux. Elle est également nécessaire à la synthèse des hormones sexuelles et à la production des neurotransmetteurs. Elle présente aussi un effet hypocholestérolémiant (par stimulation de la protéine lipase ou par inhibition de la lipolyse médiée par l'AMP cyclique dans le tissu adipeux).

Dans un mode de réalisation particulier, la composition selon l'invention comprend de la vitamine A, de la vitamine B1, de la vitamine B9, de la vitamine C, de la vitamine E, de la vitamine F et de la vitamine PP.

Par ailleurs, dans un mode préféré de réalisation, la composition de l'invention comprend en outre une huile animale, notamment une huile de poisson, en particulier une huile de poisson des mers froides (oleum pisci mare fresca). Cette huile est riche en acide gras oméga 3 qui diminuent le taux sanguin des triglycérides (par réduction de leur synthèse hépatique) plus particulièrement des VLDL. Les oméga 3 permettent une bonne fluidité membranaire.

D'autre part, dans un mode particulièrement préféré de mise en oeuvre, la composition de l'invention comprend en outre du coprah (*Cocos nucifera*). Le coprah est riche en acide gras et représente un régulateur intestinal.

Un objet particulier de l'invention concerne ainsi une composition comprenant de l'huile de colza, de l'huile d'olive, de l'huile de pépins de raisin(s), de l'huile d'onagre, une huile de poisson des mers froides, du Coprah, du sodium, du magnésium, du calcium, du zinc, du fer, un (extrait de) levure(s) Saccharomyces cerevisiae, de préférence enrichie en sélénium, des extraits végétaux de criste marine, d'ail, de Palmaria palmata (Dulse), de Chondrus crispus (Carragaeen), de Fucus vesiculosus (Varech vésiculeux), de Shiitake (mycelium) et de vigne, de la vitamine A, de la vitamine B1, de la vitamine B9, de la vitamine C, de la vitamine E, de la vitamine F et de la vitamine PP.

Selon les familles d'ingrédients, les quantités préférentielles de la composition nutraceutique et/ou alimentaire selon l'invention sont déterminées pour :
- huiles végétales (l'huile de colza, de l'huile d'olive, de l'huile de pépins de raisin(s), de l'huile d'onagre) : 28µg à 280µg/100g ou 100ml
- oligoéléments : minéraux (sodium, magnésium, calcium) et métaux (zinc et fer) : 40µg à 400µg/100g ou 100ml
- levures ou extraits de levure(s) Saccharomyces cerevisiae, de préférence enrichie en sélénium : 28µg à 280µg/100g ou 100ml
- champignons ou extraits de champignons (Shiitake mycelium): 28µg à 280µg/100g ou 100ml
- algues marines ou leurs extraits Palmaria palmata (Dulse), Chondrus crispus (Carragaeen) et Fucus vesiculosus (Varech vésiculeux): 24µg à 240µg/100g ou 100ml
- huile de poisson des mers froides et Coprah : 28µg à 280µg/100g ou 100ml
- extraits végétaux de plantes (criste marine, d'ail et de vigne): 28µg à 280µg/100g ou 100ml
- vitamines (A, B1, B9, C, E, F et PP) : 32µg à 320µg/100g ou 100ml,
en combinaison avec un ou plusieurs phytostérols, stanols ou leurs mélanges ainsi qu'un excipient pharmaceutique autorisé.

L'invention peut être mise en oeuvre chez tout Mammifère, en particulier chez les humains, adultes, âgés ou enfants

La dose de la composition nutraceutique et/ou alimentaire à administrer, à ingérer ou à appliquer à un individu peut être soumise à des variations dépendant de cet individu (âge, sexe, état de santé etc).. Il appartient aux professionnels de la santé et autres spécialistes d'ajuster cette dose en fonction des paramètres individuels à prendre en compte.

Comme indiqué précédemment, la composition nutraceutique et/ou alimentaire selon l'invention présente des propriétés avantageuses dans la régulation du métabolisme des lipides chez l'Homme ou l'Animal.

En particulier un objet de l'invention concerne une méthode pour le métabolisme des lipides chez un sujet, comprenant l'administration, l'application ou l'ingestion d'une composition nutraceutique et/ou alimentaire telle que définie dans la présente invention. Cette administration peut être réalisée de manière séquentielle (composition LIPISTASE + phytostérols/stanols) ou concomitante (composition nutraceutique et/ou alimentaire).

On pourra également utiliser la composition nutraceutique et/ou alimentaire comme médicament.

Un objet particulier de la présente invention est l'utilisation de la composition nutraceutique et/ou alimentaire, pour la préparation d'un additif alimentaire destiné à la régulation de l'homéostasie lipidique ainsi qu'à la prévention du syndrome métabolique, des risques de cancers, des maladies cardiovasculaires, de la dyslipidémie, de l'hypertension, de la progression de l'athérosclérose, de l'obésité, de l'hyperglycémie, de la dégénérescence maculaire du diabétique, des maladies neuro dégénérative chez l'Homme et l'Animal.

Par ailleurs, la composition nutraceutique et/ou alimentaire selon l'invention peut être utilisée pour la préparation d'un médicament destiné au traitement et/ou à la prévention du syndrome métabolique, des risques de cancers, des maladies cardiovasculaires, de la dyslipidémie, de l'hypertension, de la progression de l'athérosclérose, de l'obésité, de l'hyperglycémie, de la dégénérescence maculaire du diabétique, des maladies neuro dégénérative ainsi que la régulation de l'homéostasie lipidique chez l'Homme et l'Animal.

Le "syndrome métabolique" correspond à l'ensemble de perturbations métaboliques (hypertension, hypertriglycéridémie, hypoHDL-cholestérolémie, hyperglycémie et obésité) qui prédisposent fortement à la progression vers un diabète de type 2, des maladies cardio-vasculaires (athérosclérose, maladies coronariennes), des maladies cérébrovasculaires (AVC et dégénérescence centrale), des désordres endocrinaux (infertilité, impuissance), des cancers...
- Des concentrations élevées sont nécessaires pour obtenir des effets significatifs sur les profils lipidiques lors de l'administration de phytostérols et/ou stanols (PS). La composition LIPISTASE réduit significativement les paramètres lipidiques avec des mécanismes d'action différents de ceux des PS (absorption intestinale de cholestérol). Ainsi l'addition de ces deux mécanismes d'action par une co-administration de la composition LIPISTASE avec les PS (composition nutraceutique et/ou alimentaire selon l'invention) permet de ***réduire les quantités de PS*** administrées pour obtenir un ***meilleur effet sur les lipides.*** De plus, **la** ***fréquence des administrations*** de PS peut être réduite à une seule avec le maintien des résultats lipidiques. Enfin, en réduisant les quantités de PS administrées, on diminue les risques d'interférences intestinales drogues thérapeutiques / PS mais aussi les conséquences sur l'absorption intestinale.
- Dans certaines expérimentations animales, l'administration de PS tend à augmenter les triglycérides plasmatiques. La composition LIPISTASE tend à reverser cet effet et ainsi ***potentialise l'activité des PS*** sur l'homéostasie lipidique chez les patients présentant des ***dyslipidémies.***
- Aucun effet évident sur le poids corporel n'a été rapporté dans la littérature. La composition LIPISTASE ajoutée aux PS (composition nutraceutique et/ou alimentaire selon l'invention) permet aux animaux de perdre significativement du poids. Ainsi l'administration chez l'Homme de la composition nutraceutique et/ou alimentaire selon l'invention permet la ***prévention du syndrome métabolique*** chez les patients jeunes et adultes.
- Les PS diminuent significativement la concentration plasmatique des carotènes. En parallèle, des taux faibles de caroténoïdes sont associés à un risque important de maladies chroniques, comme les maladies cardiovasculaires, certains cancers et la dégénérescence maculaire.

Les effets anti thrombotiques et antioxydants observés avec la composition nutraceutique et/ou alimentaire selon l'invention permettent de ralentir de manière inattendue ***l'apoptose des cellules endothéliales,*** de stimuler la ***fonctionnalité des péricytes,*** d'améliorer la ***microcirculation*** et la ***pression sanguine*** et également d'optimiser le ***flux sanguin*** nutritif vers les tissus périphériques.

La composition LIPISTASE (via adiponectine) agit partiellement en activant l'AMP kinase. Le suppresseur tumoral LKB1 est un régulateur de l'AMP kinase et la stimulation de cette activité peut exercer un effet anti-tumoral.

Prenant en considération ces résultats, il a été montré que la composition nutraceutique et/ou alimentaire selon l'invention permet d'abaisser ***les risques de cancers*,** de ***maladies cardiovasculaires dans le syndrome métabolique*** avancé et prévient **la** ***dégénérescence maculaire*** observée chez le ***patient diabétique.***

De plus les PS, n'ayant pas d'effet significatif rapporté chez l'Homme sur l'athérosclérose, gagnent ainsi en puissance en association avec la composition LIPISTASE et ainsi la composition nutraceutique et/ou alimentaire selon l'invention montre des effets plus puissants contre la ***progression de l'athérosclérose.***

- La composition LIPISTASE peut augmenter un certain nombre de gènes dans le cerveau (ApoE, ABCA1, HMG-CoA réductase...). Les stérols quant à eux, sont supposés être des activateurs naturels des LXRs, régulateurs de l'homéostasie du cholestérol. La composition nutraceutique et/ou alimentaire selon l'invention apporte une modulation plus soutenue du métabolisme du cholestérol et des acides gras et joue un rôle important dans les ***maladies neuro dégénératives*,** comme par exemple la ***maladie d'Alzheimer.***

L'invention est décrite plus en détail par les exemples ci-après. D'autres aspects et avantages de la présente invention apparaîtront à la lecture des exemples, qui doivent être considérés comme illustratifs et non limitatifs.

### EXEMPLES

### Etude chez l'animal

### Exemple 1 : Protocole de préparation

Cinq ou six souris mâles C57bl6J (8 semaines d'âge) provenant de l'élevage Janvier (Le Genest Saint Isle ; France), sont réparties dans des cages et hébergées dans une pièce dont l'hygrométrie et la température (23±0.5°C) sont contrôlées. Elles sont soumises à un cycle de lumière 12/12 h (lumière à 7h). Pendant une semaine d'adaptation, les animaux ont accès libre à l'eau de boisson et à la nourriture puis ils sont randomisés en 5 groupes de 12 animaux en prenant comme critères de sélection le poids, la triglycéridémie et la cholestérolémie.
Les différents groupes constitués sont :
- un groupe témoin recevant une diète enrichie en cholestérol (voir composition en tableau 1),
- un groupe traité par la référence, l'atorvastatine, à 10mg/kg/jour,
- un groupe recevant la Lipistase à la concentration de 125ng/kg/jour,
- un groupe PS (mélange de phytostérols et stanols) à la dose totale de 1.2g/kg/jour,
- un groupe association de la Lipistase et des phytostérols.

Tous les produits (référence, produits en étude et association de produits) sont administrés dans la diète enrichie en cholestérol et l'administration aux animaux dure trois mois. La Lipistase se présentant sous forme de suspension d'huile de soja, tous les régimes reçoivent le même volume (1.8% P/P) d'huile de soja neutre que celui nécessaire pour apporter la quantité voulue de Lipistase.

**Tableau 1: Composition de la nourriture enrichie en cholestérol « Western Diet » (U8958 version 8, Safe, Augis, France)**

| | |
|---|---|
| Caséine | 19.5% |
| PM 205B Safe | 7% |
| PV 200 Safe | 1% |
| Malto-dextrine | 10% |
| Saccharose | 31% |
| Dextrine de mais | 5.0% |
| Methionine (DL) | 0.3% |
| Beurre laitier | 21% |
| Cellulose | 5.05% |
| Cholestérol | 0.15% |

L'évaluation du poids et de la prise alimentaire sont réalisés une fois par semaine. Toutes les 4 semaines un prélèvement sanguin (sinus rétro orbitaire) permet de suivre l'évolution de la cholestérolémie, de la triglycéridémie et de la glycémie.

L'euthanasie est réalisée au bout de 3 mois de traitement (12 semaines). Un bilan sanguin est réalisé et les paramètres suivants sont mesurés sur un automate Konelab (Thermo Fisher, France): cholestérol total (Thermo Fisher, 981813), triglycérides (Thermo Fisher, 981786), glucose (Thermo Fisher, 981780), ALAT (Thermo Fisher, 981769), ALP (Thermo Fisher, 981771), ASAT (Thermo Fisher, 981771).

La détermination des profils lipoprotéiques est réalisée par FPLC (Synelvia, France) après deux et trois mois de traitement, les quantités de cholestérol et de triglycérides sont mesurées dans les différentes fractions (HDL, LDL et VLDL).

Les dosages de l'insulinémie (Mercodia, Suède, ref 10-1150-10), de l'adiponectine (Biocat, Allemagne, ref K1002-1) et de la thyroxine (Genway, USA, ref 40-101-325036) sont réalisés après 12 semaines de traitement.

Les animaux sont sacrifiés par dislocation cervicale ; le foie, les muscles (gastrocnémien et soleus), le coeur sont rapidement disséqués, pesés et congelés dans de l'azote liquide puis stockés à -80°C. Les différents dépôts de tissus adipeux (sous cutané, péri rénal, inguinal et mésentérique) sont prélevés et pesés pour calculer l'index d'adiposité.

Les études de morphométrie du tissu adipeux sont réalisées sur 10 coupes de chaque tissu (sous cutané et périrénal) de tous les animaux (n=12). Pour chaque coupe, 100 adipocytes sont mesurés (périmètres et aires) et les valeurs obtenues sont traitées par le logiciel de morphométrie Morpho-Pro (Exploranova).

Les études histologiques du foie ont été faites sur 6 animaux de chaque lot pris au hasard dans leur groupe. Les lobes médians des foies ont été prélevés et fixés dans du formol puis inclus. 30 coupes sont gardées par animal et sont marquées aux huiles rouges pour visualiser les accumulations lipidiques.

Toutes les données sont présentées avec les moyennes et leurs esm (écart standard à la moyenne). Les tests statistiques utilisés sont une analyse de variance à un facteur suivi d'un test de Bonferroni ; les différences à p<0.05 sont considérées comme significatives. Dans certains cas, les tests de Grubb ou de Dixon sont réalisés pour identifier et exclure des valeurs aberrantes à p=0.05.

### Exemple 2 : Mise en évidence de la synergie de l'association Lipistase-phytostérols/stanols (PS) sur le poids corporel et la modification de la répartition lipidique dans l'organisme : prévention du surpoids et de l'obésité

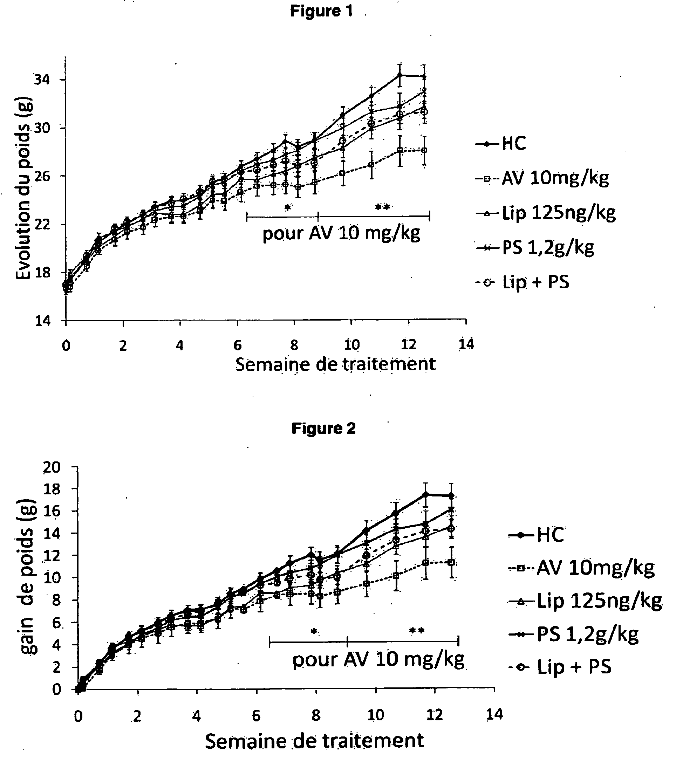

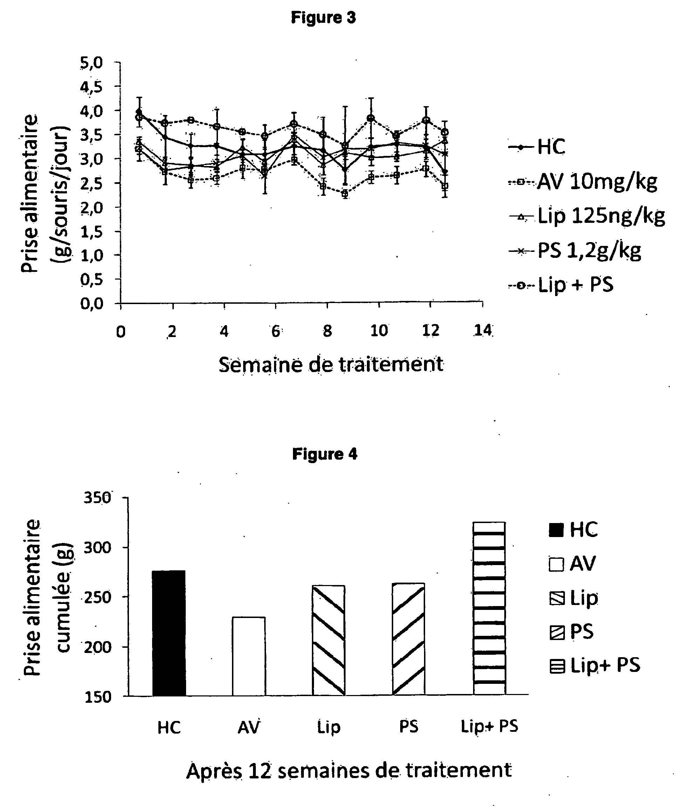

La ***figure 1*** montre l'évolution du poids corporel des souris quelque soit le traitement appliqué. Seule la référence présente un poids significativement inférieur aux poids des animaux contrôles. Cependant sur la ***figure 2*** exprimant l'évolution du gain de poids, il apparait nettement que la Lipistase tend à réduire le gain de poids et lorsqu'elle est associée aux PS qui n'ont pas cette tendance, elle garde cet effet protecteur contre le gain de poids. L'effet de l'atorvastatine sur la réduction du gain de poids est consécutif à une réduction de la prise alimentaire **(*****figure 3*****)** alors qu'il n'en est pas de même ni pour la Lipistase seule ni pour l'association Lipistase - PS. Ainsi, si l'on considère la prise alimentaire cumulée rapportée sur la ***figure 4*****,** les animaux recevant l'association Lipistase-PS consomment 323g alors que ceux recevant les PS (263g) et la Lipistase (260g) ont une consommation très proche de la valeur ingérée par les animaux contrôles (275g). La consommation de nourriture induite par l'atorvastatine est réduite à 229g. Ces constatations nous laissent penser que l'association Lipistase - PS induit un mécanisme d'action différent de celui stimulé par les composés seuls. En effet, la prise alimentaire supérieure aux autres groupes et le poids réduit observés pour le groupe d'association conduisent à une dépense énergétique plus importante qu'il faut démontrer par calorimétrie indirecte avec une modification de l'utilisation des nutriments dans l'augmentation de cette dépense énergétique (béta oxydation, catabolisme de glucose...)

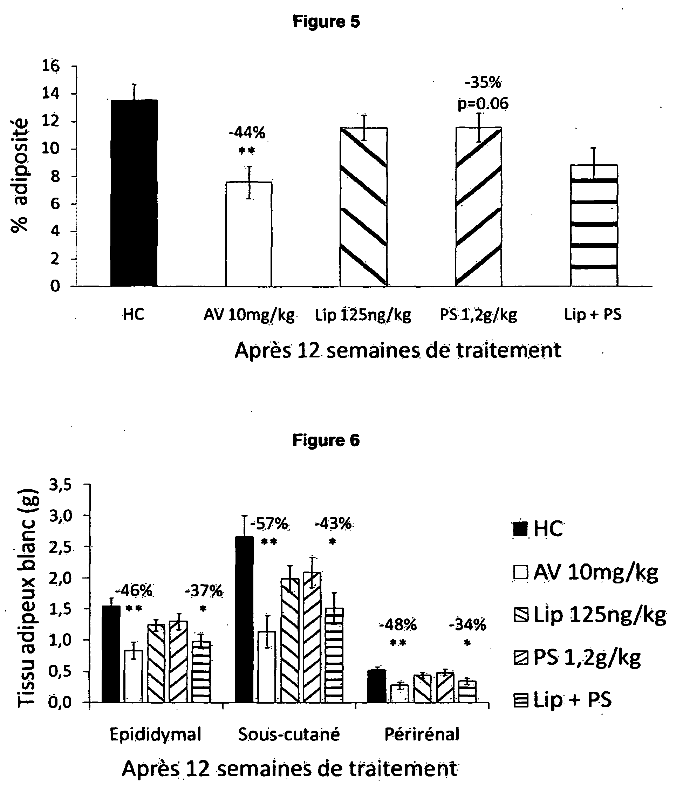

Ces résultats sont à rapprocher de ceux obtenus pour le tissu adipeux. L'index d'adiposité est rapporté sur la ***figure 5******.*** Il est montré très nettement une réduction de cet indice avec une perte de poids due au traitement avec l'atorvastatine mais également avec l'association Lipistase - PS (-35%, p=0.06) alors que les deux compositions seules sont moins efficaces (-14%, ns). Sur la **figure 6****,** seuls les traitements avec l'atorvastatine et l'association Lipistase - PS induisent des modifications significatives de la répartition du tissu adipeux blanc, avec des réductions importantes aussi bien des tissus adipeux profond [(périrénal), (respectivement -48% et -34%)] que superficiels [sous-cutanée (respectivement -57% et -43%) et épididymal (respectivement -46% et-37%)].

Ainsi la présente association selon l'invention ***prévient le surpoids*** induit par le régime enrichi en graisse et en cholestérol, similaire à celui observé.

### Exemple 3 : Mise en évidence de la synergie de l'association Lipistase-phytosterols/stanols sur les capacités sécrétrices du tissu adipeux : prévention de l'inflammation tissulaire et préservation de la fonction endocrine

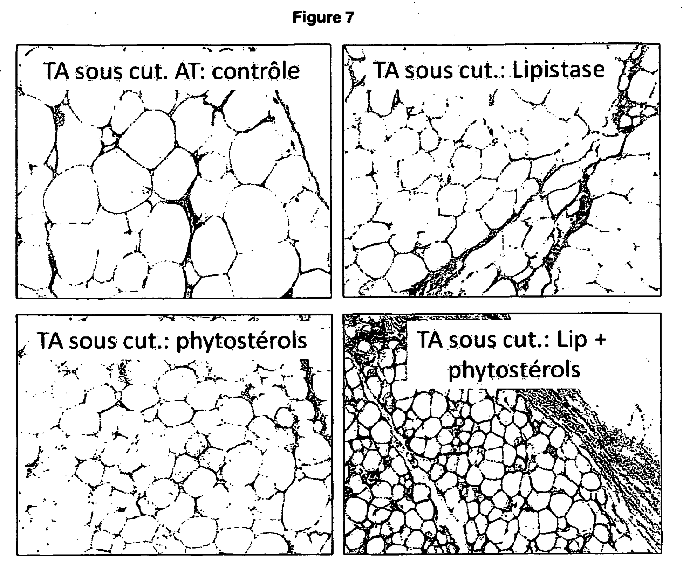

Sur la *figure* 7 sont représentées des coupes de tissu adipeux sous cutané sur des animaux ayant reçus tous les traitements. La morphologie de ces tissus présente des différences notables, plus particulièrement chez les animaux traités par l'association Lipistase - PS avec de nombreuses petites cellules alors que sur les coupes des animaux contrôles, les adipocytes sont de très larges cellules.

Les coupes des tissus adipeux des animaux contrôles présentent des infiltrations de macrophages entre les adipocytes, ces infiltrations ne se remarquent pas sur les coupes provenant des animaux traités par l'association Lipistase - PS.

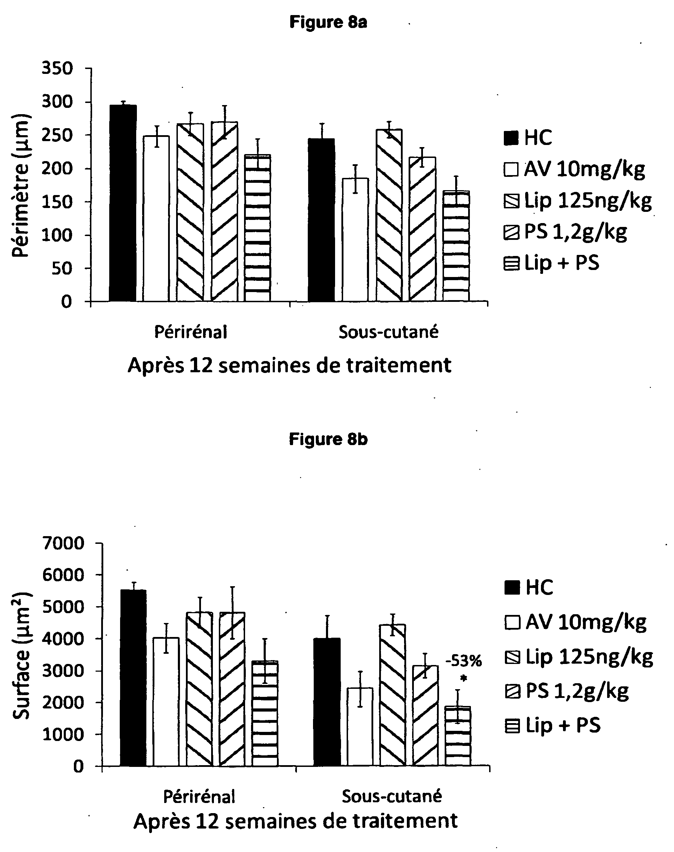

De plus, si l'on considère les données morpho métriques rapportés sur les ***figures 8a et 8b******,*** seule l'association Lipistase - PS induit des modifications significatives dans le tissu adipeux sous cutané présentant des adipocytes de faibles périmètres et de petites surfaces avec des réductions respectives de -32% et -53%.

Il est bien décrit dans la littérature que les adipocytes larges sont représentatifs d'un état d'inflammation chronique, les taux de sécrétions de cytokines telles qu'IL6, IL8 et MCP1 sont élevés et la sécrétion d'adiponectine est faible (Cole et al, 2010 ; Gustafson, 2010)

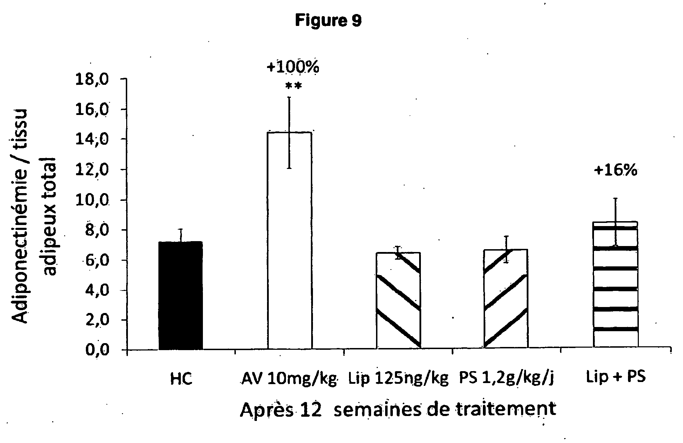

Ainsi sur la ***figure 9******,*** l'index de sécrétion d'adiponectine par le tissu adipeux est significativement élevé par la référence et, avec une échelle moindre, par l'association Lipistase - PS avec une augmentation de 16% alors que la masse du tissu adipeux diminue en moyenne de 39% (comme démontré figure 5).

De toutes ces mesures et constatations, il est nettement démontré que l'association de la composition Lipistase et des PS ***réduit le développement de la masse adipeuse tout en gardant la fonctionnalité endocrine*** du tissu adipeux en termes de sécrétion d'adiponectine voire en l'augmentant. De plus, l'association permet de ***limiter le phénomène inflammatoire*** dans ce même tissu adipeux, en s'opposant ainsi au développement du syndrome métabolique.

### Exemple 4 : Mise en évidence de la synergie de l'association Lipistase - phytosterols sur la régulation de l'homéostasie lipidique

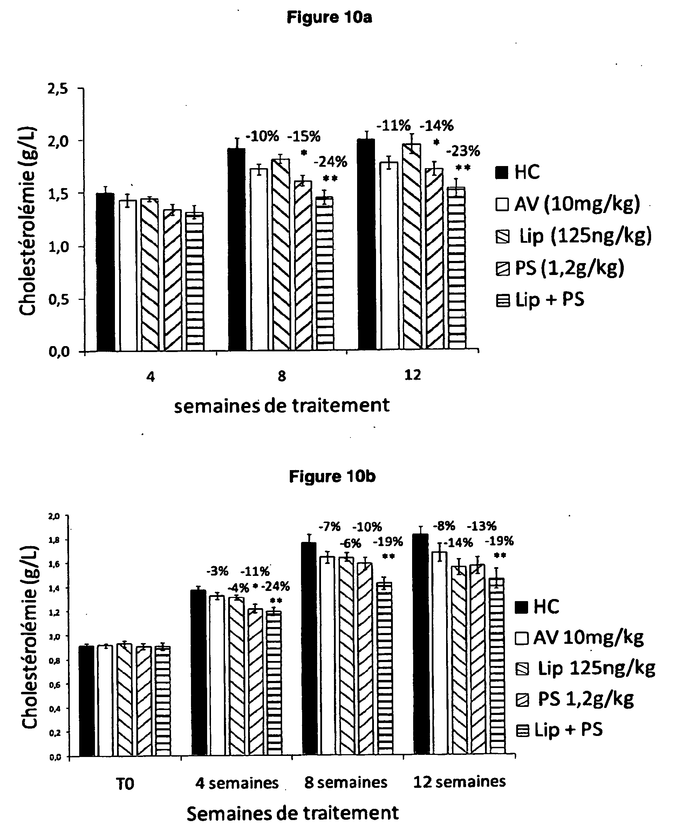

L'évolution de la cholestérolémie est rapportée sur **la** ***figure 10a*** pour les animaux non à jeun et sur la ***figure 10b*** pour les animaux ayant subi une période de jeûne de 4h. Si l'on considère les résultats obtenus lorsque les animaux ne sont pas à jeun (figure 10a), la Lipistase ne montre pas d'effet sur le cholestérol dans ce cas particulier alors que les PS diminuent significativement la cholestérolémie dès 8 semaines de traitement (-15%, *). Cependant lorsque les PS sont associés à la composition Lipistase les effets observés sur la réduction de cholestérol sont significativement plus marqués pour atteindre -24% de réduction après 8 semaines de traitement et s'y stabiliser même après 3 mois de traitement.

Lorsque les animaux sont soumis à un jeûne de 4 heures (figure 10b) les effets des PS sur le cholestérol sont beaucoup moins marqués (-11%, * à 4 semaines ; -10% ns à 8 semaines et -13% ns à 12 semaines). Concernant la composition Lipistase, les effets observés sur le cholestérol évoluent dans le temps (-4% ns à 4 semaines ; -6% à 8 semaines et -14% ns à 12 semaines) et sont comparables après 12 semaines de traitement à ceux observés avec le traitement PS. Lorsque l'association Lipistase - PS est donnée aux animaux, ceux-ci montrent une réduction significative de cholestérol dès le premier mois de traitement (-13%, *) qui s'affirme et se stabilise au deuxième et troisième mois de traitement à -19% (*).

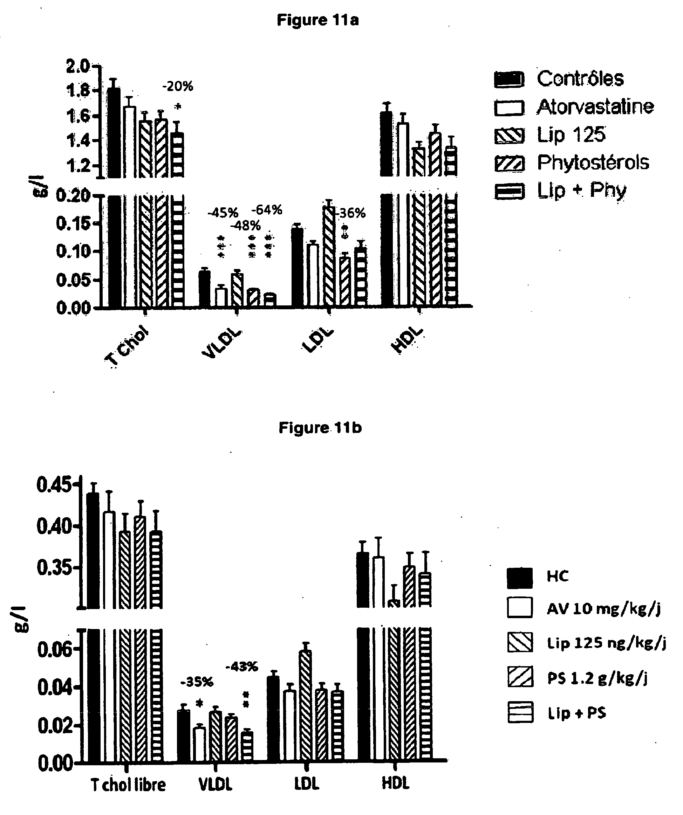

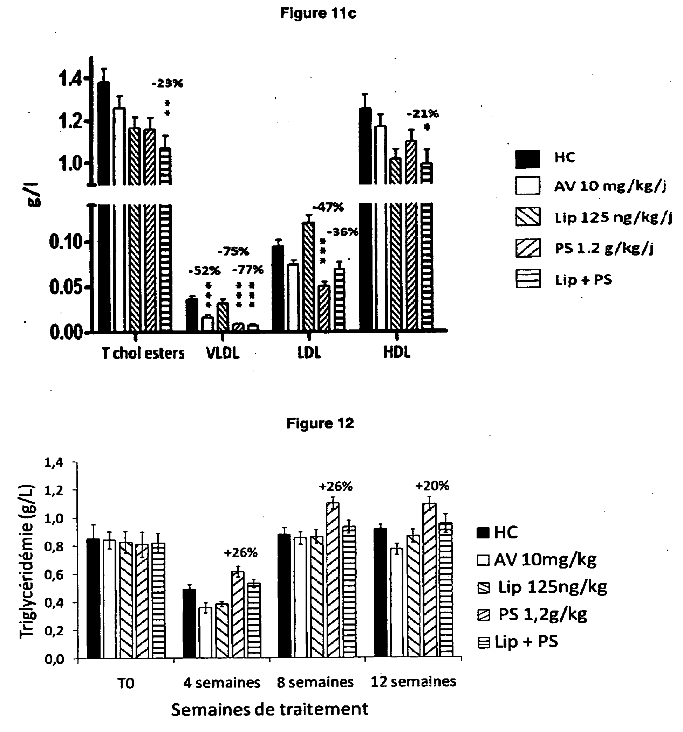

Si l'on s'attache à la répartition du cholestérol total dans les différentes lipoprotéines, on constate que les effets les plus marqués ressortent avec l'association Lipistase - PS - 64% sur les VLDL (figure ***11a*****),** alors que les PS seuls n'induisaient qu'une réduction de -48% dans cette fraction. L'association est également très efficace sur la réduction du cholestérol libre (le plus athérogène) dans les VLDL avec une réduction de -43%, l'atorvastatine n'atteignant que -35% alors que la Lipistase seul et les PS seuls n'ont pas d'effets significatifs **(*****figure 11b*****)*.*** Enfin, il apparait nettement que l'association potentialise la réduction des esters de cholestérol **(*****figure 11c*****)** dans toutes les lipoprotéines avec des réductions de -77% dans les VLDL, -36% dans les LDL et -21% dans les HDL.

Les résultats obtenus sur la triglycéridémie pour des animaux soumis à un jeûne de 4h, ***figure 12******,*** montrent un effet dans ce modèle de souris de stimulation du taux de triglycérides par les PS dès le premier mois de traitement avec une augmentation de 26%, cet effet restant stable pendant toute la durée du traitement. Cette observation doit être reliée aux capacités LXRs agonistes (Chuu *et al,* 2007) des PS ; ces agonistes sont connus pour induire la production de triglycérides chez les rongeurs. Cependant, lorsque la Lipistase est administrée en même temps que les PS, cet effet stimulateur de la triglycéridémie est contré puisque les valeurs obtenues pour l'association sont tout à fait comparables à celles des animaux contrôles.

Ainsi, l'association permet une potentialisation de ***l'effet régulateur sur la cholestérolémie,*** d'une part dans le temps en permettant le contrôle plus tôt que les composés pris séparément et d'autre part en intensité, en maintenant fortement une réduction plus marquée que celle observée avec les PS seuls et la Lipistase seule. De plus, en réduisant l'accumulation des différentes fractions de cholestérol aussi bien dans les LDL que les VLDL, l'association peut être un bon élément de ***prévention contre le développement de l'athérosclérose.*** Les effets inverses sur les triglycérides montrent une ***amélioration de l'innocuité et de la tolérance de l'association*** Lipistase - PS versus les PS seuls en plus de la ***régulation de l'homéostasie des lipides.***

### Exemple 5 : Mise en évidence de la synergie de l'association Lipistase - phytosterols (PS) sur la prévention de l'insulino-résistance

Les animaux nourris avec des diètes enrichies en graisses et en cholestérol développent progressivement un syndrome métabolique et cet effet est rapporté dans de nombreuses publications dont notamment les plus récentes Wuesst et al (2009), Bie et al (2010) et Chen et al (2010). Ce développement se caractérise, entre autre, par une augmentation de la glycémie et de l'insulinémie, traduisant ainsi l'installation progressive de l'insensibilité des tissus périphériques.

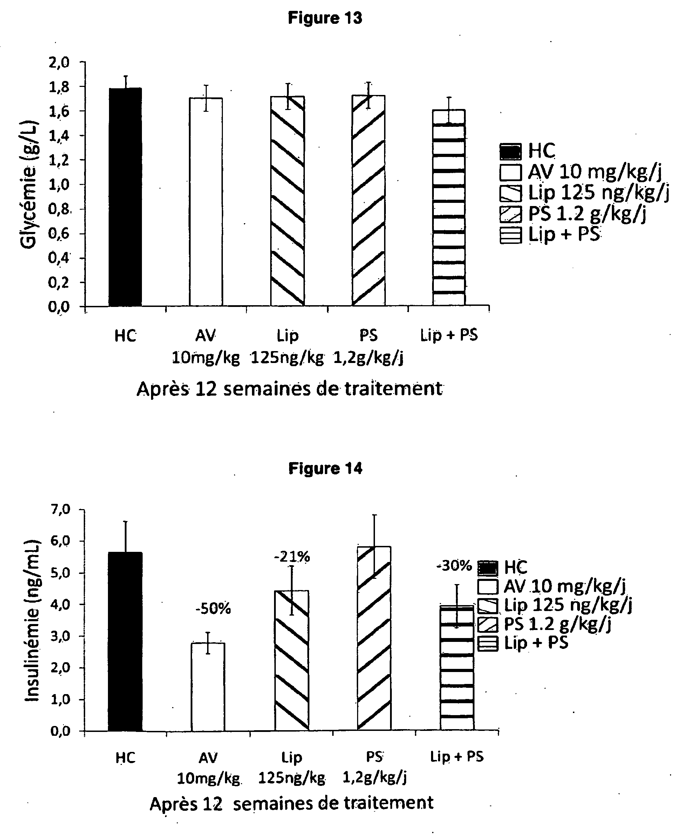

***Figure 13 et figure 14*** sont rapportées respectivement les valeurs de la glycémie (g/l) et de l'insulinémie (ng/ml). La moyenne des glycémies du groupe témoin sont de 1.78 ± 0.10 g/l. Les traitements ne présentent pas de différences significatives mais les glycémies des animaux du groupe association Lipistase-PS diminuent avec une moyenne de 1.60 ± 0.07g/l.

Si l'on considère l'insulinémie mesurée sur tous les animaux traités, l'atorvastatine diminue les insulinémie de -50% réduisant la valeur du taux circulant à 2.79 ±0.33 ng/ml versus 5.64 ± 0.99 ng/ml pour les animaux contrôles. Le traitement Lipistase seul induit une réduction plus modéré de -21% (valeur de 4.43 ±0.76 ng/ml) tandis que l'association Lipistase - PS semble plus puissante avec une réduction de -30% (valeur de 3.93 ±0.68 ng/ml). Les PS sont dépourvus d'effet aussi bien sur la glycémie que sur l'insulinémie.

Il apparait nettement que l'atorvastatine réduit l'insulino résistance puisque un taux réduit de 50% semble suffisant pour maintenir la glycémie au même niveau que celui des animaux témoins. La Lipistase réduit plus faiblement cette insulino résistance périphérique avec une réduction modérée de l'insulinémie (-21%) sans modification notable de la glycémie. En revanche l'association de la Lipistase et des PS induit une meilleure sensibilité des tissus périphériques puisque ce traitement non seulement réduit de -30% les insulinémies mais également les glycémies. Cela traduit donc un effet de potentialisation sur la capacité des tissus périphériques à consommer le glucose circulant. L'association Lipistase - PS permet donc ***d'améliorer la sensibilité périphérique au glucose et de prévenir l'insulino-résistance induite*** par le régime gras.

### Exemple 6 : Mise en évidence de la synergie de l'association Lipistase-phytosterols (PS) sur la prévention de la stéatose hépatique

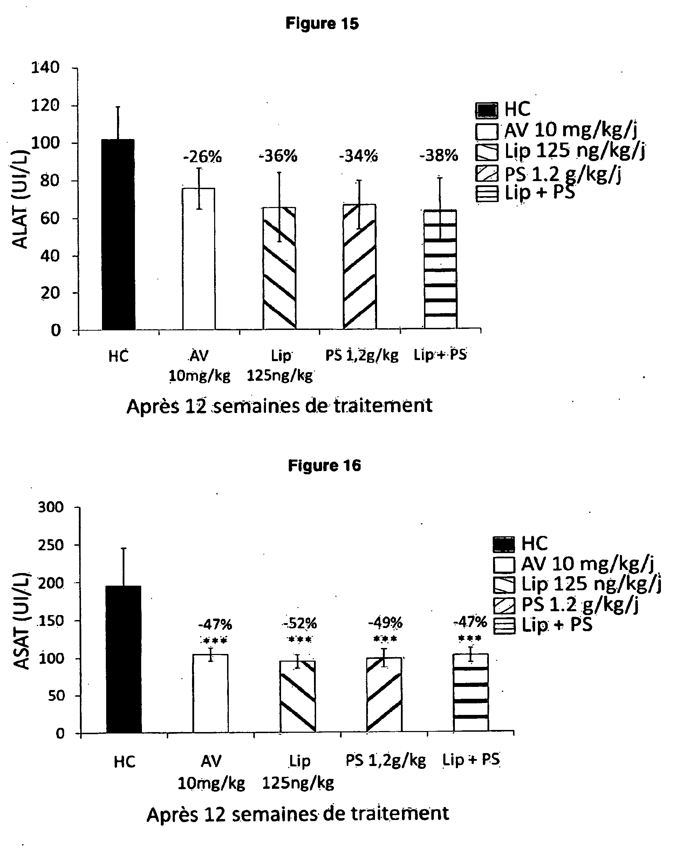

Outre l'insulino-résistance, une consommation de diète enrichie en graisse induit une stéatose hépatique (essentiellement surcharge en triglycérides). Les paramètres plasmatiques traduisant une « souffrance » hépatique sont les ASAT et les ALAT qui dans notre expérimentation sont élevées chez les contrôles. Avec les différents traitements, les valeurs sont efficacement réduites et de façon non différentielle entre les 4 traitements (en moyenne -30% pour les ALAT et -50% pour les ASAT) ***figures 15 et 16******.***

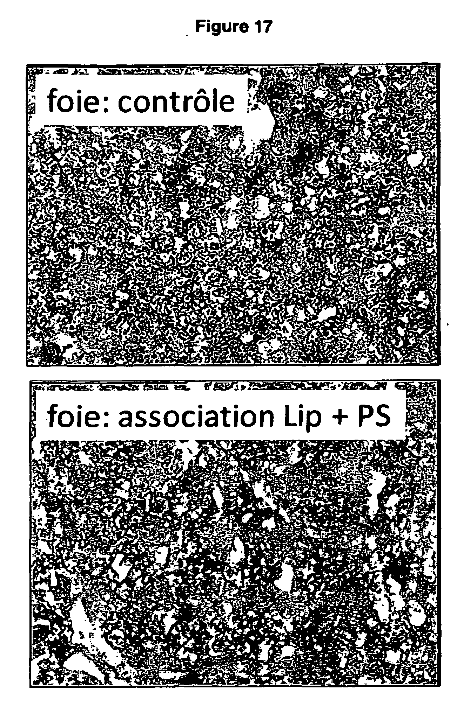

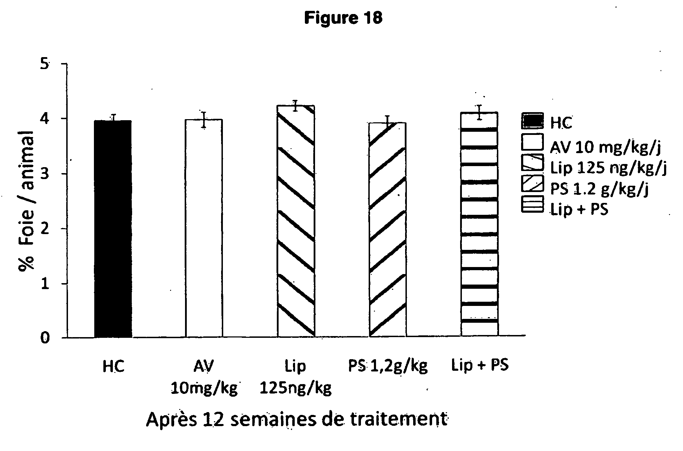

Sur la ***figure 17******,*** des coupes de foie sont rapportées après coloration aux huiles rouges pour mettre en évidence les accumulations de lipides dans les hépatocytes. Ces accumulations apparaissent sous forme de petites granulations noires qui sont très nettement visibles sur la photo montrant une coupe de foie d'animaux contrôles. Quelles que soient les coupes observées chez les animaux traités par l'association Lipistase - PS, aucun animal ne présente d'accumulation lipidique sur les coupes, alors que certains animaux en montrent avec la Lipistase seule ou les PS seuls en traitement. Ces effets sont intéressants même si le ratio poids du foie / poids corporel n'est pas modifié ***figure 18******.***

Ainsi l'association de la Lipistase avec les PS permet de ***prévenir le développement de la stéatose dans le foie*** tout en gardant une ***tolérance hépatique.***

### REFRENCES

Clifton PM, Noakes M, Ross D, Fassoulakis A, Cehun M, Nestel P, "High dietary intake of phystosterol esters decreases carotenoids and increases plasma plant sterol levels with no additional cholesterol lowering", J Lipid Res, 2004, 45, 1493-1499.
Denke MA, "Lack of efficacy of low-dose sitostanol therapy as an adjunct to a cholesterol-lowering diet in men with moderate hypercholesterolemia", Am J Clin Nutr, 1995, 61, 392-396.
Katan MB, Grundy SM, jones P, Law M, Miettinen T, Paoletti R, "Efficacy and safety of plants stanols and sterols in the management of blood cholesterol levels", Mayo Clin Proc, 2003, 78, 965-078.
Trautwein EA, Duchateau GS, Lin YG, Mel'nikov SM, Molhuizen HOF, Ntanios FY, "Proposed mechanisms of cholesterol-lowering action ofplant sterols" Eur J Lipid Sci Technol, 2003, 105, 171-185.
Bie J, Zhao B, Song J, Ghosh S ; « Improved insulin sensitivity in high-cholesterol fed LDLR-/- mice with macrophage-specific transgenic expression of cholesteryl ester hydrolase : role of macrophage inflammation and infiltration into adipose tissue » ; J Biol Chem ; 2010 ; Feb 26.
Chen X, Yu QQ, Zhu YH, Bi Y, Sun WP, Liang H, Cai MY, He XY, Weng JP ; « insulin therapy stimulates lipid synthesis and improve endocrine fonctions of adipocytes in dietary obese C57bl/6 mice » ; Acta Pharmacol Sin ; 2010 ; 31(3) :341-346.
Chuu CP, Kokontis JM, Hiipakka RA, Liao S, « modulation of LXR signaling as novel therapy for prostate cancer » ; J Biomed Sci, 2007, 14, 543-553.
Cole BK, Keller SR, Wu R, Carter JD, Nadler JL, Nunemaker CS, « Valsartan protects pancreatic islets and adipose tissue from the inflammatory and metabolic consequences of a high fat diet in mice » ; Hypertension ; 2010 ; 55(3), 715-721.
Gustafson B, « Adipose tissue, inflammation and atherosclerosis », J Atheroscler Thromb, 2010 Feb 3
Wueest S, Rapold RA, Rytka JM, Schoenle EJ, Konrad D, « Basal lipolysis, not the degree of insulin resistance, differentiates large from small isolated adipocytes in high-fat fad mice » ; Diabetologia ; 2009 ; 52 :541-546.

## Revendications

1. Composition nutraceutique et/ou alimentaire destinée à la régulation du métabolisme des lipides chez l'Homme ou l'Animal **caractérisé en ce que** ladite composition comprend par 100g/100ml la combinaison de:
• 7µg à 700µg d'au moins deux huiles végétales sélectionnées parmi l'huile de colza, l'huile d'olive, l'huile de pépins de raisin et l'huile d'onagre,
• 10µg à 1000µg de minéraux chargés positivement choisis parmi le sodium, le magnésium et le calcium,
• 10µg à 1000µg de métaux choisis parmi le zinc et le fer,
• 7µg à 700µg de levures ou extraits de levure provenant du genre *Saccharomyces cerevisiae,* **caractérisé en ce que** ces dites levures ou extraits de levure sont enrichies en sélénium,
• 7µg à 700µg de champignons ou d'extraits de champignon shiitaké (mycelium),
• 6µg à 600µg d'au moins deux extraits végétaux de plante choisis parmi la criste marine, l'ail et la vigne,
• 8µg à 800µg d'au moins une vitamine choisie parmi les vitamines A, B1, B9, C, E, F et PP,
• 7µg à 700µg d'huile animale et de coprah (Cocos nucifera),
• 6µg à 600µg d'au moins une algue choisie parmi Palmaria palmata (Dulse), Chondrus crispus (Carragaeen) et Fucus vesiculosus (Varech vésiculeux), en combinaison avec un ou plusieurs phytostérols, stanols ou leurs mélanges ainsi qu'un excipient pharmaceutique autorisé.

2. Composition nutraceutique et/ou alimentaire destinée à la régulation du métabolisme des lipides chez l'Homme ou l'Animal selon la revendication 1, **caractérisée en ce que** l'huile animale consiste en de l'huile de poisson des mers froides (Oleum Pisci mare fresca).

3. Composition nutraceutique et/ou alimentaire destinée à la régulation du métabolisme des lipides chez l'Homme ou l'Animal selon la revendication 1 ou 2, **caractérisée en ce que** ladite composition contient au moins deux vitamines choisies parmi les vitamines A, B1, B9, C, E, F et PP

4. Composition nutraceutique et/ou alimentaire selon l'une des revendications précédentes, **caractérisée en ce qu'**elle comprend par 100g/100 ml de :
• 7µg à 700µg d'huile de colza, de l'huile d'olive, de l'huile de pépins de raisin(s), de l'huile d'onagre,
• 10µg à 1000µg de sodium, de magnésium et de calcium,
• 10µg à 1000µg de zinc et le fer,
• 7µg à 700µg de levures ou extraits de levure de Saccharomyces cerevisiae, enrichis en sélénium,
• 7µg à 700µg de mycélium ou d'extrait de mycélium de Shiitake,
• 6µg à 600µg de criste marine, d'ail et de vigne,
• 8µg à 800µg de vitamines A, B1, B9, C, E, F et PP
• 7µg à 700µg d'huile de poisson des mers froides et de coprah,
• 6µg à 600µg de Palmaria palmata (Dulse), Chondrus crispus (Carragaeen) et Fucus vesiculosus (Varech vésiculeux),
en combinaison avec un ou plusieurs phytostérols, stanols ou leurs mélanges ainsi qu'un excipient pharmaceutique autorisé.

5. Composition nutraceutique et/ou alimentaire selon l'une quelconque des revendications précédentes, **caractérisée en ce qu'**elle comprend en outre des excipients ou additifs, tels que des agents édulcorants, stabilisants, conservateurs, colorants, émulsifiants ou gélifiants, exhausteurs de goût, acidifiants, arômes.

6. Composition nutraceutique et/ou alimentaire selon l'une quelconque des revendications précédentes, **caractérisée en ce que** lesdits phytostérols, stanols (PS) utilisés sont sélectionnés parmi le groupe comprenant: le brassicastérol, campestérol, campestanol, stigmasterol, beta-sitosterol, beta sitostanol, D5 avenastérol, D7 stigmasténol, D7Avenastérol ou leur combinaisons et mélanges.

7. Composition nutraceutique et/ou alimentaire selon l'une quelconque des revendications précédentes, **caractérisée en ce qu'**elle se présente sous forme solide, liquide, huile, de gel, filmstrips, pâte, poudre ou gomme.

8. Composition nutraceutique et/ou alimentaire selon l'une quelconque des revendications précédentes, **caractérisée en ce qu'**elle est adaptée à une administration orale.

9. Composition nutraceutique et/ou alimentaire selon l'une quelconque des revendications précédentes, en tant que médicament.

10. Utilisation de la composition nutraceutique et/ou alimentaire selon l'une quelconque des revendications 1 à 8, pour la préparation d'un additif alimentaire destiné à la régulation de l'homéostasie lipidique ainsi qu'à la prévention du syndrome métabolique, des risques de cancers, des maladies cardiovasculaires, de la dyslipidémie, de l'hypertension, de la progression de l'athérosclérose, de l'obésité, de l'hyperglycémie, de la dégénérescence maculaire du diabétique, des maladies neuro dégénérative chez l'Homme et l'Animal.

11. Utilisation de la composition nutraceutique et/ou alimentaire selon l'une quelconque des revendications 1 à 8, pour la fabrication d'un médicament destiné au traitement et/ou à la prévention du syndrome métabolique, des risques de cancers, des maladies cardiovasculaires, de la dyslipidémie, de l'hypertension, de la progression de l'athérosclérose, de l'obésité, de l'hyperglycémie, de la dégénérescence maculaire du diabétique, des maladies neuro-dégénérative ainsi que la régulation de l'homéostasie lipidique chez l'Homme et l'Animal.

## Patentansprüche

1. Nutraceutical-Zusammensetzung und / oder Lebensmittel für die Regulation des Lipidstoffwechsels bei Menschen oder Tieren, **dadurch gekennzeichnet, dass** die Zusammensetzung für 100g / 100ml die Kombination aufweist von:
- 7 µg bis 700 µg von mindestens zwei Pflanzenölen, die aus Öl, Rapsöl, Olivenöl, Trauben Kernöl und Nachtkerze,
- 10 µg bis 1000 µg von positiv geladenen Mineralien aus Natrium, Magnesium und Calcium,
- 10 µg bis 1000 µg ausgewählt Metalle aus Zink-und Eisenmetalle,
- 7 µg bis 700 µg Hefe oder Hefe-Extrakte der Gattung *Saccharomyces cerevisiae,* **dadurch gekennzeichnet, dass** die genannten Hefen oder Hefe-Extrakte mit Selen angereichert sind,
- 7 µg bis 700 µg Pilz oder Extrakte aus Shiitake-Pilz (Myzel),
- 6 µg bis 600 µg von mindestens zwei ausgewählten Pflanzenextrakten aus Bazillenkraut, Knoblauch und Wein,
- 8 µg bis 800 µg von mindestens einem Vitamin, ausgewählt aus den Vitaminen A, B1, B9, C, E, F und PP,
- 7 µg bis 700 µg Tierisches Öl und Kokosnuss (Cocos nucifera),
- 6 µg bis 600 µg von mindestens einer Alge ausgewählt aus Palmaria palmata (Dulse), Chondrus crispus (Carragaeen) und Blasentang (Wrack) in Kombination mit einem oder mehreren Phytosterole, stanols oder deren Mischungen sowie ein zugelassenes Pharma-H ilfsstoff.

2. Nutraceutical-Zusammensetzung und / oder Lebensmittel für die Regulation des Lipidstoffwechsels bei Menschen oder Tieren nach Anspruch 1, **dadurch gekennzeichnet, dass** das Tierische Öl aus Kaltwasser Fischöl (*Oleum Pisci mare fresca)* besteht.

3. Nutraceutical-Zusammensetzung und / oder Lebensmittel für die Regulation des Lipidstoffwechsels bei Menschen oder Tieren nach Anspruch 1 oder 2, **dadurch gekennzeichnet, dass** die Zusammensetzung mindestens zwei ausgewählte Vitamine aus den Vitaminen A, B1, B9, C, E, F und PP enthält.

4. Nutraceutical-Zusammensetzung und / oder Lebensmittel nach einem der vorhergehenden Ansprüche, **dadurch gekennzeichnet, dass** sie pro 100g/100ml umfasst:
- 7 µg bis 700 µg Rapsöl, Olivenöl, Traube(n) Kernöl, Nachtkerzen Öl,
- 10 µg bis 1000 µg Natrium, Magnesium und Calcium,
- 10 µg bis 1000 µg Zink und Eisen
- 7 µg bis 700 µg Hefe oder Hefeextrakt aus Saccharomyces cerevisiae mit Selen angereichert,
- 7 µg bis 700 µg Myzel oder Myzel-Extrakt aus Shiitake,
- 6 µg bis 600 µg Bazillenkraut, Knoblauch und Wein,
- 8 µg bis 800 µg der Vitamine A, B1, B9, C, E, F und PP,
- 7 µg bis 700 µg Kaltwasser Fischöl und Kopra,
- 6 µg bis 600 µg Palmaria palmata (Dulse), Chondrus crispus (Carragaeen) und Fucus (Blasentang),
in Kombination mit einem oder mehreren Phytosterolen, stanols, oder Mischungen davon und ein zugelassener pharmazeutischer Hilfsstoff.

5. Nutraceutical-Zusammensetzung und / oder Lebensmittel nach einem der vorhergehenden Ansprüche, **dadurch gekennzeichnet, dass** es ferner Additive oder Hilfsstoffe, wie Süßstoffe, Stabilisatoren, Konservierungsmittel, Farbstoffe, Gelbildner oder Emulgatoren, Geschmacksverstärker, Säuerungsmittel, Geschmacksstoffe enthält.

6. Nutraceutical-Zusammensetzung und / oder Lebensmittel nach einem der vorhergehenden Ansprüche, **dadurch gekennzeichnet, dass** die Phytosterole, Phytostanole (PS) aus der Gruppe umfassend: Brassicasterol, Campesterol, Campestanol, Stigmasterol, beta-Sitosterol, D5 Avenasterol, D7 stigmastenol, D7 Avenasterol oder Kombinationen und Mischungen ausgewählt sind.

7. Nutraceutical-Zusammensetzung und / oder Lebensmittel nach einem der vorhergehenden Ansprüche, **dadurch gekennzeichnet, dass** sie in fester, flüssiger, Öl, Gel, Filmstreifen, Paste, Puder oder Kaugummi Form besteht.

8. Nutraceutical-Zusammensetzung und / oder Lebensmittel nach einem der vorhergehenden Ansprüche, **dadurch gekennzeichnet, dass** sie für die orale Verabreichung geeignet ist.

9. Nutraceutical-Zusammensetzung und / oder Lebensmittel nach einem der vorhergehenden Ansprüche als Arzneimittel.

10. Verwendung der Nutraceutical-Zusammensetzung und / oder Lebensmittel nach einem der Ansprüche 1 bis 8, zur Herstellung eines Lebensmittelzusatzstoff der zur Regulierung der Lipid-Homöostase und der Prävention von Metabolischem Syndrom, Risiken von Krebs, kardiovaskulärer Erkrankung, Dyslipidämie, Bluthochdruck, Fortschreiten der Arteriosklerose, Fettleibigkeit, Hyperglykämie, diabetischen Makuladegeneration, neurodegenerativen Erkrankungen bei Mensch und Tier bestimmt ist.

11. Verwendung der Nutraceutical-Zusammensetzung und / oder Lebensmittel nach einem der Ansprüche 1 bis 8, zur Herstellung eines Medikaments der zur Behandlung und / oder Prävention von Metabolischem Syndrom, das Risiko von Krebs, kardiovaskulären Erkrankungen, Dyslipidämie, Bluthochdruck, Fortschreiten der Arteriosklerose, Fettleibigkeit, Hyperglykämie, diabetischen Makuladegeneration, neuro-degenerative und Regel Lipidhomöostase Krankheiten bei Mensch und Tier bestimmt ist.

## Claims

1. Nutraceutical composition and / or food for the regulation of lipid metabolism in humans or for Animal **characterized in that** said composition comprises for 100g / 100ml the combination of:
- 7 µg to 700 µg of at least two vegetable oils selected from oil, rapeseed oil, olive oil, grape seed oil and evening primrose,
- 10 µg to 1000 µg of positively charged minerals selected from sodium, magnesium and calcium
- 10 µg to 1000 µg of metals selected from zinc and iron metals,
- 7 µg to 700 µg of yeast or yeast extracts from the genus *Saccharomyces cerevisiae,* **characterized in that** the said yeasts or yeast extracts are enriched in selenium,
- 7 µg to 700 µg of mushrooms or extracts from shiitake mushroom (mycelium),
- 6 µg to 600 µg of at least two plant extracts selected from plant samphire, garlic and vine,
- 8 µg to 800 µg of at least one vitamin selected from vitamins A, B1, B9, C, E, F and PP,
- 7 µg to 700 µg of animal oil and copra (Cocos nucifera),
- 6 µg to 600 µg of at least one alga selected from Palmaria palmata (Dulse), Chondrus crispus (Carrageen) and bladder wrack (wrack), in combination with one or more phytosterols, stanols or their mixtures as well as an authorized pharmaceutical excipient.

2. The nutraceutical composition and / or food for the regulation of lipid metabolism in humans or animal according to claim 1, **characterized in that** the animal oil consists of cold water fish (*Oleum Pisci mare fresca*).

3. The nutraceutical composition and / or food for the regulation of lipid metabolism in humans or animal according to claim 1 or 2, **characterized in that** said composition contains at least two vitamins chosen from vitamins A, B1, B9, C, E, F and PP.

4. The nutraceutical composition and / or food composition according to any one of the preceding claims, **characterized in that** it comprises per 100g/100 ml:
- 7 µg to 700 µg of rapeseed oil, olive oil, grape seed(s) oil, oil of evening primrose,
- 10 µg to 1000 µg of sodium, magnesium and calcium,
- 10 µg to 1000 µg of zinc and iron
- 7 µg to 700 µg of yeast or yeast extracts from *Saccharomyces cerevisiae* enriched with selenium,
- 7 µg to 700 µg of mycelium or mycelium extract of Shiitake,
- 6 µg to 600 µg of sea fennel, garlic and vine,
- 8 µg to 800 µg of vitamins A, B1, B9, C, E, F and PP ,
- 7 µg to 700 µg of cold water fish oil and copra,
- 6 µg to 600 µg of Palmaria palmata (Dulse), Chondrus crispus (Carrageen) and Fucus vesiculosus (wrack),
in combination with one or more phytosterols, stanols or mixtures thereof as well as an authorized pharmaceutical excipient.

5. The nutraceutical and / or food composition according to any of the preceding claims, **characterized in that** it further comprises additives or excipients, such as composition sweetening agents, stabilizers, preservatives, dyes, gelling agents or emulsifiers, flavor enhancers, acidifiers, flavorings.

6. The nutraceutical composition and / or food composition according to any one of the preceding claims, **characterized in that** said used phytosterols, stanols (PS) are selected from the group comprising: brassicasterol, campesterol, campestanol, stigmasterol, beta sitosterol, D5 avenasterol, D7 stigmastenol, D7 Avenasterol or combinations and mixtures thereof.

7. The nutraceutical composition and / or food composition according to any preceding claims, **characterized in that** it is in the form of a solid, liquid, oil, gel, filmstrips, paste, powder or gum.

8. The nutraceutical composition and / or food composition according to any preceding claims, **characterized in that** it is adapted for oral administration.

9. The nutraceutical composition and / or food composition according to any one of the preceding claims, as a medicament.

10. Use of the nutraceutical composition and / or food composition according to any one of claims 1 to 8, for the preparation of a food additive for the regulation of lipid homeostasis and the prevention of metabolic syndrome, risks of cancer, cardiovascular disease, dyslipidemia, hypertension, progression of atherosclerosis, obesity, hyperglycemia, diabetic macular degeneration, neurodegenerative diseases of humans and animals.

11. The use of the nutraceutical composition and / or food composition according to any one of claims 1 to 8, for the manufacture of a medicament for the treatment and / or prevention of metabolic syndrome, the risk of cancer, cardiovascular diseases, dyslipidemia, hypertension, progression of atherosclerosis, obesity, hyperglycemia, diabetic macular degeneration, neuro-degenerative and regulating lipid homeostasis diseases of humans and animals.
